# EUROPEAN PATENT APPLICATION

(11) **EP 0 574 118 A1**
(43) Date of publication of application: **15.12.1993**
(21) Application number: 93303016.5
(22) Date of filing: 20.04.1993
(51) Int. Cl.: C09B 57/00, C07D 493/04

(54) **Polycyclic dyes**

(30) Priority: 08.06.1992 GB 9212095
(71) Applicant: ZENECA LIMITED, London W1Y 6LN (GB)
(72) Inventor: Kenyon, Ronald Wynford, Failsworth, Manchester M35 0GW (GB); James, Mark Robert, Constable Lee Park, Rawtenstall BB4 8JY (GB)
(74) Representative: Giles, David Eric

(57) **Abstract**

A benzodifuranone dye carrying at least one thiazolyl group, the dye may be of Formula (3) or Formula (5): 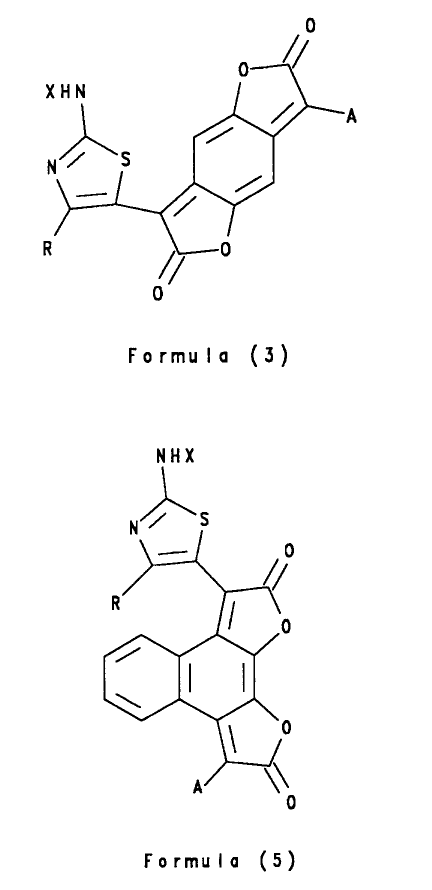
in which
- R: is -H or alkyl;
- X: is -H, alkyl or alkylcarbonyl;
- A: is a phenyl group which is unsubstituted by from one to three substituents or
- A: is a group of Formula (4):

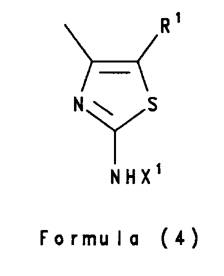

wherein:
- R¹: is -H or alkyl; and
- X¹: is -H, alkyl or alkylcarbonyl.

The benzodifuranone dyes are useful for the coloration of synthetic textile material such as polyester.

## Description

The present invention relates to polycyclic dyes.

According to the present invention there is provided a benzodifuranone dye carrying at least one thiazolyl group.

The benzodifuranone dye preferably carries at least one thiazol-4-yl group or at least one thiazol-5-yl group, more preferably at least one thiazol-5-yl group and especially two thiazol-5-yl groups. The thiazolyl groups are preferably substituted by substituents independently selected from C₁₋₆-alkyl, -NH₂, -NHC₁₋₆-alkyl, -N(C₁₋₆-alkyl)₂, -NHCOC₁₋₆-alkyl.

The benzodifuranone dyes are preferably of Formula (1): 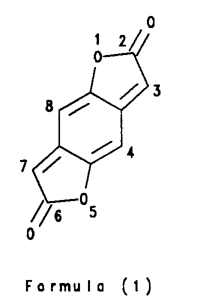
where the thiazolyl group or groups is preferably attached to the 3- or to the 3- and 7-positions or of Formula (2): 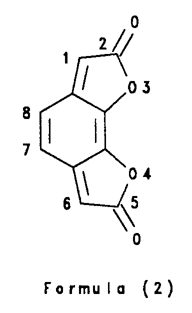
in which the thiazolyl group or groups is preferably attached to the 1- or to the 1- and 6-positions.

In dyes of Formula (1) which carry one thiazolyl group in the 3-position the 7-position is preferably occupied by an optionally substituted phenyl group. In dyes of Formula (2) which carry one thiazolyl group in the 1-position the 6-position is preferably occupied by an optionally substituted phenyl group.

A preferred sub group of benzodifuranone dyes of Formula (1) are of the Formula (3): 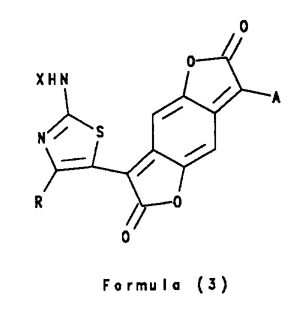
wherein:
- R: is -H or alkyl;
- X: is -H, alkyl or alkylcarbonyl;
- A: is a phenyl group which is unsubstituted or substituted by from one to three substituents; or
- A: is a group of the Formula (4):

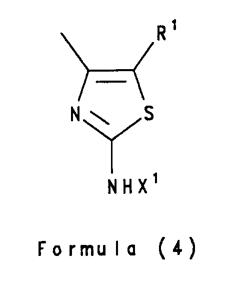

wherein:
- R¹: is -H or alkyl; and
- X¹: is -H, alkyl or alkylcarbonyl.
Where any one of R, R¹, X and X¹ is alkyl it preferably contains from 1 to 10 carbon atoms, more preferably from 1 to 6 carbon atoms and especially from 1 to 4 carbon atoms. When X or X¹ is alkylcarbonyl it is preferably C₁₋₆-alkylcarbonyl, more preferably C₁₋₄-alkylcarbonyl and especially -COCH₃, -COC₂H₅ and -COCH(CH₃)₂. The alkyl groups and the alkyl part of the alkylcarbonyl groups may be straight or branched chain alkyl and may be substituted by substituents selected from phenyl, halogen such as -F, -Cl and -Br, nitro, cyano, hydroxy, amino, alkylamino, dialkylamino, alkoxy, alkoxyalkoxy, alkoxycarbonyl, alkylcarbonyl, alkoxyalkoxycarbonyl, alkylcarbonyloxy and alkylcarbonylamino in which each alkyl is preferably C₁₋₄-alkyl and each alkoxy is preferably C₁₋₄-alkoxy.

When A is a substituted phenyl group the substituents are preferably in the 4-position, or in the 3- and 4-positions, or in the 3-, 4- and 5-positions. Suitable substituents for the phenyl group may be independently selected from any of those substituents described above for the alkyl groups represented by R, R¹, X and X¹ and preferably selected from C₁₋₄-alkoxy, amino, C₁₋₄-alkylamino and C₁₋₄-alkyl.

A preferred sub group of benzodifuranone dyes of Formula (2) are of the Formula (5): 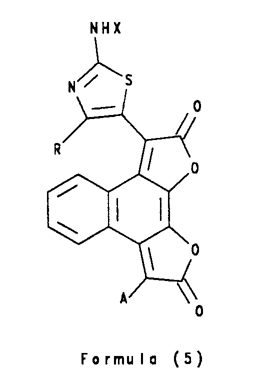
wherein X, R and A are as hereinbefore defined.

The benzodifuranone dyes may be conveniently prepared by reaction of a dihydroxybenzene with a substituted acetic acid to give an intermediate benzofuranone which may be reacted with a further equivalent of the same or a different substituted acetic acid to give the benzodifuranone dye. The reactions may be carried out at a temperature of from 25°C to 100°C in a liquid medium such as acetic acid which may optionally contain sulphuric acid. The benzodifuranone dyes may be conveniently isolated by diluting the reaction mixture with water, adding sodium hydroxide solution and collection of the product by filtration or by diluting with aqueous buffer (pH 7.0) and collecting the product by filtration.

Substituted acetic acids which carry thiazolyl substituents may be prepared by reacting a thiazole with glyoxylic acid in aqueous media, maintained at pH 10.5 by the addition of an aqueous base such as sodium hydroxide solution at an elevated temperature, the substituted acetic acids may be isolated by acidifying the reaction mixture with an aqueous acid such as hydrochloric acid and filtration.

The dyes of the present invention are useful for the coloration of synthetic textile material, especially polyesters.

The invention is further illustrated by the following examples:

### Example 1

### Preparation of 3-(2-acetamido-4-methylthiazol-5-yl)-7-(2-isobutyramido-4-methylthiazol-5-yl)-2,6-dioxo-2,6-dihydrobenzofuran[1:2-b, 4:5-b']difuran

### i) 2-(2-Acetamido-4-methylthiazol-5-yl)-2-hydroxyacetic acid

A rigorously stirred mixture of 2-acetamido-4-methylthiazole (24.6g, 0.16 mol) and glyoxylic acid (50% aqueous solution, 46.7g, 0.31 mol) in water (280cm³) was made alkaline to pH 10.5 by the dropwise addition of 48% sodium hydroxide solution. This mixture was stirred at 50-60°C while maintaining the pH at 10.5 by the dropwise addition of 48% sodium hydroxide solution until the suspended solid went into solution. The reaction mixture was cooled and filtered, the filtrates were acidified to pH 3 with hydrochloric acid (32%), the product filtered off, washed with water (4x100cm³) and dried at 60°C to give the 2-(2-acetamido-4-methylthiazol-5-yl)-2-hydroxyacetic acid (35.2g). m.p. 190°C darkens, 208°C decomposed.

### ii) 3-(2-Acetamido-4-methylthiazol-5-yl)-5-hydroxy-2-oxo-2,3-dihydrobenzo[1:2-b]furan

A mixture of 2-(2-acetamido-4-methylthiazol-5-yl)-2-hydroxyacetic acid (51.6g, 0.23 mol) hydroquinone (42.0g, 0.38 mol) and 5% v/v sulphuric acid in acetic acid (500cm³) was stirred at 70°C for 24 hours. The cooled solution was poured into a stirred mixture of ice and water (1.5dm³) and filtered. Ice (500g) was added to the filtrate and the pH adjusted to 3.75 by the dropwise addition of 48% sodium hydroxide solution. The product was collected by filtration, washed with water (4x100cm³) and air dried to give 3-(2-acetamido-4-methylthiazol-5-yl)-5-hydroxy-2-oxo-2,3-dihydrobenzo[1:2-b]furan (27.8g), m.p. >240°C.

### iii) 2-Iso-butyramido-4-methylthiazole

A mixture of 2-amino-4-methylthiazole (34.8g, 0.31 mol) and iso-butyric anhydride (76cm³) was stirred at 0°C for 15 minutes and at ambient temperature for 16 hours. The reaction mixture was poured into a mixture of ice and water (500cm³) and made alkaline by adding sodium carbonate, the mixture was extracted with ethyl acetate (2x200cm³) the ethyl acetate was washed with water, dried over anhydrous magnesium sulphate and evaporated to give 2-iso-butyramido-4-methylthiazole (52.1g).

### iv) 2-(2-Iso-butyramido-4-methylthiazol-5-yl)-2-hydroxyacetic acid

A vigorously stirred mixture of 2-iso-butyramido-4-methylthiazole (52.2g, 0.23 mol) and glyoxylic acid (50% aqueous solution, 82.6g, 0.55 mol) in water (500cm³) was made alkaline to pH 10.5 by the dropwise addition of 48% sodium hydroxide solution. The mixture was stirred at 50-60°C while maintaining the pH at 10.5 by the dropwise addition of 48% sodium hydroxide solution until the suspended solid went into solution. The reaction mixture was cooled and filtered, the filtrates were acidified to pH 3 with hydrochloric acid (32%), the product filtered off and washed with water (4x100cm³) and dried at 60°C to give 2-2(-iso-butyramido-4-methylthiazol-5-yl)-2-hydroxyacetic acid (65g) m.p. 190°C darkens, 201°C decomposes.

### v) 3-(2-Acetamido-4-methylthiazol-5-yl)-7-(2-iso-butyramido-4-methylthiazol-5-yl)-2,6-dioxo-2,6-dihydrobenzo[1:2-b, 4:5-b']difuran

Six equal portions of 2-(2-iso-butyramido-4-methylthiazol-5-yl)-2-hydroxyacetic acid (7.56g, 29.4 mol) were added to a vigorously stirred mixture of 3-(2-acetamido-4-methylthiazol-5-yl)-5-hydroxy-2-oxo-2,3-dihydrobenzo[1:2-b]furan (5.95g, 19.6 mol) in 5% v/v sulphuric acid in acetic acid (100cm³) at 75°C for 3 days. The reaction mixture was cooled and poured into a stirred mixture of saturated phosphate (pH 7.0) buffer and ice (700cm³) and the precipitated product was isolated by filtration and dried under vacuum, this crude product was purified by liquid chromatography from silica using ethyl acetate as eluent, evaporation of the ethyl acetate gave 2-2(-iso-butyramido-4-methylthiazol-5-yl)-2-hydroxyacetic acid (1.1g) λₘₐₓ (ethylacetate) = 580nm.

### Example 2

### Preparation of 3,7-di(2-iso-butyramido-4-methylthiazol-5-yl)-2,6-dioxo-2,6-dihydrobenzo[1:2-b, 4:5-b']difuran

A mixture of 2-(2-iso-butyramido-4-methylthiazol-5-yl)-2-hydroxyacetic acid (26.7g, 0.10 mol), hydroquinone (19.3g, 0.18 mol) and 5% v/v sulphuric acid in acetic acid (50cm³) was stirred at 75°C for 1 day. The cooled solution was poured into a stirred mixture of saturated phosphate (pH 7.0) buffer and ice (700cm³) the precipitated product was isolated by filtration and dried to 50°C, this crude product was purified by liquid chromatography from silica using ethylacetate as eluent. Evaporation of the ethylacetate gave 3,7-di(2-iso-butyramido-4-methylthiazol-5-yl)-2,6-dioxo-2,6-dihydrobenzo[1:2-b, 4:5-b']difuran m.p. 266(dec)°C, λₘₐₓ (ethylacetate) = 578 nm.

### Example 3

### Preparation of 3-(2-acetamido-4-methylthiazol-5-yl)-7-(4-methyl-2-propionamidothiazol-5-yl)-2,6-dioxo-2,6-dihydrobenzo[1:2-b, 4:5-b'] difuran

The method of Example 1v) was followed except that 4.46g instead of 5.95g of 3-(2-acetamido-4-methylthiazol-5-yl)-5-hydroxy-2-oxo-2,3-dihydrobenzo[1:2-b]furan was used and 4.46g of 2-(4-methyl-2-propionamidothiazol-5-yl)-2-hydroxyacetic acid was used in place of 2-(2-iso-butramido-4-methylthiazol-5-yl)-2-hydroxyacetic acid. 3-(2-Acetamido-4-methylthiazol-5-yl-7-(4-methyl-2-propionamidothiazol-5-yl)-3,6-dioxo-2,6-dihydrobenzo[1:2-b, 4:5-b']difuran (0.6g) was obtained, λₘₐₓ (ethylacetate) = 576 nm.

### Example 4

### Preparation of 3-(2-acetamido-4-ethylthiazol-5-yl)-7-(2-acetamido-4-methylthiazol-5-yl)-2,6-dioxo-2,6-dihydrobenzo[1:2-b, 4:5-b']difuran

### i) 2-Amino-4-ethylthiazole

Iodine (145.8g, 0.57 mol) was added to a mixture of ethyl methyl ketone (250cm³) and thiourea (87.3g, 1.15 mol) and the mixture stirred under reflux for 3 hours. The mixture was cooled and the solvent removed to leave a solid which was dissolved in boiling water (500cm³) containing carbon DY3 (5g) and filtered. The filtrates were cooled (ice) and sodium hydroxide (141g) was added before extracting the mixture with ether (3x150cm³). The combined extracts were dried over anhydrous magnesium sulphate and evaporated to give a mixture of 2-amino-4-ethylthiazole and 2-amino-4,5-dimethylthiazole (61g) as an oil.

### ii) 2-Acetamido-4-ethylthiazole

The mixture of 2-amino-4,5-dimethylthiazole and 2-amino-4-ethylthiazole (61g) from above was cooled in ice and acetic anhydride (150ml) was added. The mixture was stirred at 0°C for ½ hours and at ambient temperature for a further 3½ hours before pouring into a mixture of ice and water (700g). After stirring over night the mixture was extracted with dichloromethane (4x150cm³), washed with water (300cm³), dried over anhydrous magnesium sulphate before evaporating the solvent to leave a mixture of 2-acetamido-4-ethylthiazole and 2-acetamido-4,5-dimethylthiazole (102.9g) as an oil containing acetic acid.

### iii) 2-(2-Acetamido-4-ethylthiazol-5-yl)-2-hydroxyacetic acid

A vigorously stirred mixture of 2-acetamido-4,5-dimethylthiazole and 2-acetamido-4-ethylthiazole (46g, 70%) and glyoxylic acid (29g, 50% aqueous solution) in water (300cm³) was made alkaline to pH 10.5 by the dropwise addition of 48% sodium hydroxide solution. During this addition the temperature rose to approximately 50°C. The mixture was stirred at 50-55°C maintaining the pH at 10.5 by adding 48% NaOH solution as required for 5 hours and then over night at 50°C.
The mixture was cooled and the solid material was recovered by filtration and dried to give 2-acetamido-4,5-dimethylthiazole (11.88g, 62%) m.p. 135-7°C. The filtrate was acidified to pH 3.0 by the dropwise addition of concentrated hydrochloric acid and the solid filtered off, washed with water (3x100cm³) and dried under vacuum to give 2-(2-acetamido-4-ethylthiazol-5-yl)-2-hydroxyacetic acid (17.6g, 86%), m.p. 190°C darkens, 208°C decomposes.

### iv) 3-(2-Acetamido-4-ethylthiazol-5-yl)-7-(2-acetamido-4-methyl thiazol-5-yl)-2,6-dioxo-2,6-dihydrobenzo[1:2-b, 4:5-b']difuran

Six equal portions of 2-(2-acetamido-4-ethylthiazol-5-yl)-2-hydroxyacetic acid (total 8.52g, 34.9mmol) were added to a vigorously stirred mixture of 3-(2-acetamido-4-methylthiazol-5-yl)-5-hydroxy-2-oxo-2,3-dihydrobenzo[1:2-b]furan (7.08g, 23.3mmol) in 5% v/v sulphuric acid in acetic acid (140cm³) over a period of three days while maintaining the reaction temperature at 75°C. The cooled reaction mixture was poured into stirred mixture of saturated phosphate buffer (pH 7.0) and ice (700cm³), the product was isolated by filtration and dried at ambient temperature under vacuum. The product was purified by liquid chromatography from silica using ethylacetate as eluent, evaporation of the ethylacetate gave 3-(2-acetamido-4-ethylthiazol-5-yl)-7-(2-acetamido-4-methylthiazol-5-yl)-2,6-dioxo-2,6-dihydrobenzo[1:2-b, 4:5-b']difuran as a black solid (0.84g) m.p. >310°C, λₘₐₓ (ethylacetate) = 585nm, ₘₐₓ (pyridine) = 612nm.

### Example 5

### Preparation of 3-(2-amino-4-ethylthiazol-5-yl)-7-(2-amino-4-methylthiazol-5-yl)-2,6-dioxo-2,6-dihydrobenz[1:2-b, 4:5-b']difuran

A mixture of 3-(2-acetamido-4-ethylthiazol-5-yl)-7-(2-acetamido-4-methylthiazol-5-yl)-2,6-dioxo-2,6-dihydro[1:2-b, 5:6-b']difuran (0.6g, 1.17 mmol), methyl cellosolve (5cm³) and hydrochloric acid (1cm³ of 5 moldm⁻³) was stirred at 100°C for 40 minutes. The reaction mixture was cooled and poured into saturated phosphate buffer (pH 7.0), the precipitated product was isolated by filtration, washed with water (3x50cm³) and air dried to give 3-(2-amino-4-ethylthiazol-5-yl)-7-(2-amino-4-methylthiazol-5-yl)-2,6-dioxo-2,6-dihydrobenzo[1:2-b, 4:5-b']difuran (0.07g) m.p. >310°C, λₘₐₓ (ethylacetate) = 650nm.

### Example 6

### Preparation of 3-(2-amino-4-methylthiazol-5-yl)-7-(2-amino-4-propyl thiazol-5-yl)-2,6-dioxo-2,6-dihydrobenz[1:2-b, 4:5-b']difuran

### i) 2-Amino-4-propylthiazole

The method of Example 4i) was followed except that 2-pentanone (150cm³) was used in place of the ethylmethyl ketone, 99.5g instead of 145.8g of iodine and 59.6g instead of 87.3g of thiourea were used to give a mixture of 2-amino-4-propylthiazole and 2-amino-4-ethyl-5-methyl thiazole (49.4g, 89%) as an oil.

### ii) 2-Acetamido-4-propylthiazole

The method of Example 4ii) was followed except that the mixture from 6i) was used in place of the mixture from 4i) to give a mixture of 2-acetamido-4-propylthiazole and 2-acetamido-4-ethyl-5-methylthiazole (83.2g).

### iii) 2-(2-Acetamido-4-propylthiazol-5-yl)-2-hydroxyacetic acid

The method of Example 4iii) was followed except that the mixture from 6ii) was used in place of the mixture from 4ii) to give 2-acetamido-4-ethyl-5-methylthiazole (45g) m.p. 109°C and 2-(2-acetamido-4-propylthiazol-5-yl)-2-hydroxyacetic acid (23.3g, 41%) m.p. 174°C darkens, 196°C decomposes.

### Example 7

### Preparation of 3-(2-amino-4-methylthiazol-5-yl)-7-(2-amino-4-propyl thiazol-5-yl)-2,6-dioxo-2,6-dihydrobenzo[1:2-b, 4:5-b']difuran

A mixture of 3-(2-acetamido-4-methylthiazol-5-yl)-7-(2-acetamido-4-propylthaizol-5-yl)-2,6-dioxo-2,6-dihydrobenz[1:2-b, 4:5-b] difuran (0.291g, 0.56 mmol), methyl cellosolve (5cm³) and hydrochloric acid (1cm 3 of 5 moldm⁻³) was stirred at 100°C for 40 min. The cooled mixture was poured into saturated phosphate buffer (pH 7.0, 70cm³), filtered, the product was washed with water (3x50cm³) and air dried to give 3-(2-amino-4-methylthiazol-5-yl)-7-(2-amino-4-propylthiazol-5-yl)-2,6-dioxo-2,6-dihydrobenzo[1:2-b, 4:5-b']difuran (0.193g), λₘₐₓ (ethylacetate) = 640nm.

### Example 8

### Preparation of 3,7-di(2-acetamido-4-propylthiazol-5-yl)-2,6-dioxo-2,6-dihydrobenzo[1:2-b, 4:5-b']difuran

A mixture of hydroquinone (0.83g, 7.61 mmol), 2-(2-acetamido-4-propylthiazol-5-yl)-2-hydroxyacetic acid (4.91g, 19.0 mmol) and 5% v/v sulphuric acid in acetic acid (50cm³) was stirred at 75°C for 1 day. The cooled mixture was poured into stirred mixture of saturated phosphate buffer (pH 7.0) and ice (700cm³), filtered and dried at 50°C. The crude mixture was purified by soxhlet extraction into dichlorometane to give 3,7-di(2-acetamido-4-propylthiazol-5-yl)-2,6-dioxo-2,6-dihydro benzo[1:2-b, 4:5-b']difuran (0.23g m.p. >310°C, λₘₐₓ (ethylacetate) = 596nm.

### Example 9

### Preparation of 1,6-di(2-acetamido-4-propylthiazol-5-yl)-2,5-dioxo-2,5-dihydronaphtho[2:1-b, 3:4-b']difuran

A mixture of 2-(2-acetamido-4-propylthiazol-5-yl)-2-hydroxy acetic acid (4.71g, 18.4 mmol) and 2,3-dihydroxynaphthalene (1.17g, 7.33 mmol) were reacted under the conditions of Example 2 to give 1,6-di(2-acetamido-4-propylthiazol-5-yl)-2,5-dioxo-2,5-dihydronaphtho[2:1-b, 3:4-b']difuran (0.67g, 15%), m.p. >310°C, λₘₐₓ (ethylacetate) = 504nm.

### Example 10

### Preparation of 3-phenyl-7-(2-aminothiazol-5-yl)-2,6-dioxo-2,6-dihydro benzo[1:2-b, 4:5-b']difuran

### i) 3-Phenyl-5-hydroxy-2-oxo-2,3-dihydrobenzo[1:2-b]furan

A mixture of mandelic acid (162.5g), hydroquinone (200g) and sulphuric acid (600cm³ of 73%) was stirred at 85-95°C for 3 hours before cooling to ambient temperature and pouring into a mixture of ice water (3dm³). The precipitated product was isolated by filtration, washed acid free with water and dried to give 3-phenyl-5-hydroxy-2-oxo-2,3-dihydrobenzo[1:2-b]furan (232.5g) as a pale orange solid.

### ii) 2-(2-Aminothiazol-5-yl)-2-hydroxyacetic acid

A mixture of 2-aminothiazole (18.2g), water (125cm³) and glyoxylic acid monohydrate (25.1g) was stirred and the pH was adjusted to 10.5 by addition of sodium hydroxide solution.
The mixture was stirred at 40-45°C for 3 hours. The reaction mixture was cooled, carbon screened and the filtrate acidified to pH 3.0 with concentrated hydrochloric acid and cooled in an ice bath, the precipitated product was collected by filtration, washed with water and dried at 60°C to give 2-(2-aminothiazol-5-yl)-2-hydroxyacetic acid (28.8g).

### iii) 3-Phenyl-7-(2-aminothiazol-5-yl)-2,6-dioxo-2,6-dihydrobenzo [1:2-b, 4:5-b']difuran

A mixture of 3-phenyl-5-hydroxy-2-oxo-2,3-dihydrobenzo[1:2-b] furan (2.3g), 2-(2-aminothiazol-5-yl)-2-hydroxyacetic acid (1.9g) and methanesulphonic acid (10cm³) was stirred and heated at 70-75°C for 2 hours. Ammonium persulphate (2.5g) was added and the mixture stirred at 70-75°C for 1 hour the reaction mixture was cooled and added to water (200cm³), filtered, washed acid free with water and dried to give a residue (3.15g) which was extracted with pyridine (soxhlet). The pyridine was removed under vacuum to leave a residue which was washed with methanol. The residue was purified by liquid chromatography on silica using pyridine as eluent. Removal of the pyridine under vacuum left a residue which was washed with methanol and dried at 60°C to give 3-phenyl-7-(2-aminothiazol-5-yl)-2,6-dioxo-2,6- dihydrobenzo[1:2-b, 4:5-b']difuran wt (0.11g), λₘₐₓ (pyridine) = 616nm.

### Example 11

### Preparation of 3,7-di(2-aminothiazol-5-yl)-2,6-dioxo-2,6-dihydrobenzo [1:2-b, 4:5-b']difuran

A mixture of hydroquinone (1.1g), 2-(2-aminothiazol-5-yl)-2-hydroxyacetic acid (3.1g) and methanesulphonic acid (10cm³) was stirred at ambient temperature for 16 hours. Methanesulphonic acid (5cm³) and potassium persulphate (2.5g) were added. The mixture was stirred at ambient temperature for 7 hours before adding to a mixture of ice and water. The precipitated product was isolated by filtration, washed acid free with water and dried at 60°C to give 3,7-di(2-aminothiazol-5-yl)-2,6-dioxo-2,6-dihydrobenzo[1:2-b, 4:5-b']difuran (0.85g), λₘₐₓ (pyridine) = 674nm.

### Example 12

### Preparation of 3-(4-acetylamino-3-methylphenyl)-7-(2-aminothiazol-5-yl)-2,6-dioxo-2,6-dihydrobenzo[1:2-b, 4:5-b']difuran

### i) 3-(4-Acetylamino-3-methylphenyl)-5-hydroxy-2-oxo-2,3-dihydrobenzo[1:2-b]furan

A mixture of 3-methyl-4-acetylaminomandelic acid (14g), hydroquinone (6.9g), acetic acid (95cm³) and sulphuric acid (5cm³) was stirred at 80°C for 12 hours before cooling to ambinet temperature and pouring into water (1dm³). The precipitated product was isolated by filtration, washed acid-free with water and dried to give 3-(4-acetylamino-3-methylphenyl)-5-hydroxy-2-oxo-2,3-dihydrobenzo[1:2-b]furan.

### ii) 3-(4-Acetylamino-3-methylphenyl)-7-(2-aminothiazol-5-yl)-2,6-dioxo-2,6-dihydrobenzo[1:2-b, 4:5-b']difuran

A mixture of 3-(4-acetylamino-3-methylphenyl)-5-hydroxy-2-oxo-2,3-dihydrobenzo[1:2-b]furan (1.1g), 2-(2-aminothiazol-5-yl)-2-hydroxy acetic acid (0.7g) and methanesulphonic acid (10cm³) were stirred at ambient temperature for 16 hours. Ammonium persulphate (0.9g) was added and the reaction mixture stirred at ambient temperature for 3 hours before adding to water (200cm³) and filtering. The product was washed acid free with water, washed with methanol, and dried at 60°C to give 3-(4-acetylamino-3-methylphenyl)-7-(2-aminothiazol-5-yl)-2,6-dioxo-2,6-dihydrobenzo[1:2-b, 4:5-b']difuran (1.02g).

### Example 13

### Preparation of 3-(4-amino-3-methylphenyl)-7-(2-aminothiazol-5-yl)-2,6-dioxo-2,6-dihydrobenzo[1:2-b, 4:5-b']difuran

A mixture of 3-(4-acetylamino-3-methylphenyl)-7-(2-amino thiazol-5-yl)-2,6-dioxo-2,6-dihydrobenzo[1:2-b, 4:5-b']difuran (1.0g) and sulphuric acid 68% v/v (25cm³) was stirred and heated at 120-130°C for 3½ hours. The mixture was cooled by filtration, the precipitated product was collected, washed acid free with water and dried at 60°C. The product was extracted with acetone (soxhlet) and then with pyridine, the solvent were removed under vacuum and the residue washed with methanol and dried.

The residue was purified by liquid on silica using a 1:2 mixture of chloroform and pyridine as eluent removal of the solvent under vacuum gave 3-(4-amino-3-methylphenyl)-7-(2-aminothiazol-5-yl)-2,6-dioxo-2,6-dihydrobenzo[1:2-b, 4:5-b']difuran (0.01g), λₘₐₓ (pyridine) = 665nm.

### Example 14

### Preparation of 3-(4-acetylamino)-7-(2-aminothiazol-5-yl)-2,6-dioxo-2,6-dihydrobenzo[1:2-b, 4:5-b']difuran

### i) 3-(4-acetylaminophenyl)-5-hydroxy-2-oxo-2,3-dihydrobenzo [1:2-b]furan

A mixture of 4-acetylaminomandelic acid (21g), hydroquinone (11g), acetic acid (95cm³) and sulphuric acid (5cm³) was stirred at 55°C for 21 hours before cooling to ambient temperature and pouring into water (1dm³). The precipitated product was isolated by filtration, washed acid-free with water and dried to give 3-(4-acetylaminophenyl)-5-hydroxy-2-oxo-2,3-dihydrobenzo[1:2-b]furan.

### ii) 3-(4-Acetylaminophenyl)-7-(2-aminothiazol-5-yl)-2,6-dioxo-2,6-dihydrobenzo[1:2-b, 4:5-b']difuran

A mixture of 3-(4-acetylaminophenyl)-5-hydroxy-2-oxo-2,3-dihydrobenzo[1:2-b]furan (1.0g), 2-(2-aminothiazol-5-yl)-2-hydroxyacetic acid (0.7g) and methanesulphonic acid (10cm³) was stirred at ambient temperature for 16 hours. Ammonium persulphate (0.92g) was added and the mixture was stirred at ambient temperature for 3 hours before adding to water (200cm³). The mixture was stirred for 10 mins and the precipitated solid was isolated by filtration, washed acid free with water, washed with methanol and dried at 60°C to give 3-(4-acetylamino)-7-(2-aminothiazol-5-yl)-2,6-dioxo-2,6-dihydrobenzo [1:2-b, 4:5-b']difuran (1.13g).

### Example 15

### Preparation of 3-(4-aminophenyl)-7-(2-aminothiazol-5-yl)-2,6-dioxo-2,6-dihydrobenzo[1:2-b, 4:5-b']difuran

A mixture of 3-(4-acetylamino)-7-(2-aminothiazol-5-yl)-2,6-dioxo-2,6-dihydrobenzo[1:2-b, 4:5-b']difuran (1.13g) and sulphuric acid (68%, 25cm³) was stirred at 120-130°C for 5 hour.
The reaction mixture was cooled and added to water (200cm³), the precipitated solid isolated by filtration washed acid free with water and dried at 60°C. The solid was extracted into pyridine, the pyridine was removed under vacuum and the residue was washed with methanol and dried at 60°C to give 3-(4-aminophenyl)-7-(2-aminothiazol-5-yl)-2,6-dioxo-2,6-dihydrobenzo[1:2-b, 4:5-b']difuran (0.4g).

### Example 16

### Preparation of 3-(4-N-1-methylpropy-1-yl)aminophenyl)-7-(2-(N-1-methyl prop-1-yl)aminothiazol-5-yl)-2,6-dioxo-2,6-dihydrobenzo[1:2-b, 4:5-b']difuran

A mixture of 3-(4-aminophenyl)-7-(2-aminothiazol-5-yl)-2,6-dioxo-2,6-dihydrobenzo[1:2-b, 4:5-b']difuran (0.4g), methylethyl ketone (40cm³) and glacial acetic acid (8cm³) was stirred and heated to reflux Sodium borohydride (0.5g) was added and the mixture was stirred under reflux for 1 hour. Further sodium borohydride (0.5g) was added and the mixture was stirred under reflux for 1 hour, further acetic acid (2cm³) and sodium borohydride (0.5g) was added and the mixture was stirred under reflux for 45 minutes. The mixture was poured into water (300cm³), the precipitated solid was isolated by filtration, washed with water and dried. The solid was purified by liquid chromatography from silica using mixture of chloroform and ethylacetate as eluent. The chloroform and ethylacetate was removed under vacuum and the residue washed with methanol and dried to give 3-(4-N-1-methylprop-1-yl)amino phenyl)-7-(2-(N-1-methylprop-1-yl)aminothiazol-5-yl)-2,6-dioxo-2,6-dihydrobenzo[1:2-b, 4:5-b']difuran (0.015g, λₘₐₓ (chloroform) = 666nm.

### Example 17

### Preparation of 3-(4-acetylaminophenyl)-7-(2-(N-ethylamino)thiazol-5-yl)-2,6-dioxo-2,6-dihydrobenzo[1:2-b, 4:5-b']difuran

### i) 2-(2-(N-ethylamino)thiazol-5-yl)-2-hydroxyacetic acid

The procedure of Example 1i) was followed except that 2-(N-ethylamino)thiazole (2.3g) was used in place of the 2-acetamido-4-methylthiazole, 2.5g instead of 46.7g of glyoxylic acid and a 50% v/v mixture of ethanol and water (25cm³) was used in place of the water to give 2-(2-(N-ethylamino)thiazol-5-yl)-2-hydroxyacetic acid (2.55g) as an amber viscous oil.

### ii) 3-(4-acetylaminophenyl)-7-(2-(N-ethylamino)thiazol-5-yl)-2,6-dioxo-2,6-dihydrobenzo[1:2-b, 4:5-b']difuran

A mixture of 3-(4-acetylaminophenyl)-5-hydroxy-2-oxo-2,3-dihydrobenzo[1:2-b]furan (3.0g), 2-(2-(N-ethylamino)thiazol-5-yl)-2-hydroxyacetic acid (2.55g) and a 95:5 mixture of acetic and sulphuric acid (50cm³) was stirred at 75-85°C for 12 hours. Ammonium persulphate (2.7g) was added and the reaction mixture was stirred at 75-85°C for 1 hour before cooling and adding water (250cm³). The product was isolated by filtration, washed acid free with water and dried to give 3-(4-acetylaminophenyl)-7-(2-(N-ethylamino)thiazol-5-yl)-2,6-dioxo-2,6-dihydrobenzo[1:2-b, 4:5-b']difuran (3.7g).

### Example 18

### Preparation of 3-(4-aminophenyl)-7-(2-(N-ethylamino)thiazol-5-yl-2,6-dioxo-2,6-dihydrobenzo[1:2-b, 4:5-b']difuran

A mixture of 3-(4-acetylaminophenyl)-7-(2-(N-ethylamino) thiazol-5-yl)-2,6-dioxo-2,6-dihydrobenzo[1:2-b, 4:5-b']difuran (3.7g) and sulphuric acid (68%, 67cm³) was stirred and heated on a steam bath for 30 hours. The mixture was cooled and added to water (350cm³), the product was isolated by filtration, washed acid free with water and dried before extracting with pyridine. The pyridine was removed under vacuum and the residue was washed with methanol and dried at 60°C to give 3-(4-aminophenyl)-7-(2-(N-ethylamino)thiazol-5-yl-2,6-dioxo-2,6-dihydrobenzo[1:2-b, 4:5-b']difuran (0.9g), λₘₐₓ (ethylacetate) = 634nm.

### Example 19

### Preparation of 3-(4-acetylamino-3-methylphenyl)-7-(2-(N-ethylamino) thiazol-5-yl)-2,6-dioxo-2,6-dihydrobenzo[1:2-b, 4:5-b']difuran

A mixture of 2-(2-(N-ethylamino)thiazol-5-yl)-2-hydroxyacetic acid (1.85g), 3-(4-Acetylamino-3-methylphenyl)-5-hydroxy-2-oxo-2,3-dihydrobenzo[1:2-b]furan (2.75g) and a 95:5 mixture of acetic and sulphuric acids (50cm³) was stirred 75-80°C for 12 hours. Ammonium persulphate (2.0g) was added and the mixture was stirred at 75-80°C for 1 hour.
The mixture was cooled and added to water (250cm³) the precipitated solid was isolated by filtration, washed with water, washed with methanol and dried at 60°C to give 3-(4-acetylamino-3-methyl phenyl)-7-(2-(N-ethylamino)thiazol-5-yl)-2,6-dioxo-2,6-dihydrobenzo [1:2-b, 4:5-b']difuran (2.1g)

### Example 20

### Preparation of 3-(4-amino-3-methylphenyl)-7-(2-(N-ethylamino)thiazol-5-yl)-2,6-dioxo-2,6-dihydrobenzo[1:2-b, 4:5-b']difuran

A mixture of 3-(4-acetylamino-3-methylphenyl)-7-(2-(N-ethyl amino)thiazol-5-yl)-2,6-dioxo-2,6-dihydrobenzo[1:2-b, 4:5-b']difuran (2.1g) and sulphuric acid (68%, 40cm³) was stirred and heated on steam bath for 16 hours. The residue was cooled and added to water (25cm³). The product was isolated by filtration, washed acid free with water and dried at 60°C. The product was purified by extraction into a 1:1 mixture of chloroform and pyridine and by liquid chromatography from silica using a 1:1 mixture chloroform and pyridine as eluent, removal of the solvent under vacuum gave 3-(4-amino-3-methylphenyl)-7-(2-(N-ethyl amino)thiazol-5-yl)-2,6-dioxo-2,6-dihydrobenzo[1:2-b, 4:5-b']difuran, λₘₐₓ (pyridine) = 668nm.

## Claims

1. A benzodifuranone dye carrying at least one thiazolyl group.

2. A benzodifuranone dye of Formula (3): 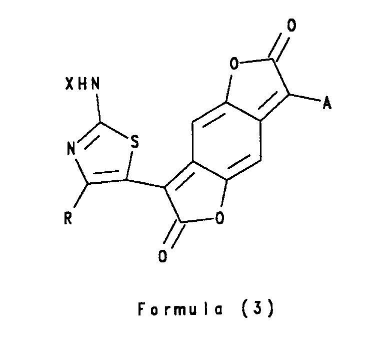 wherein:
R is -H or alkyl;
X is -H, alkyl or alkylcarbonyl;
A is a phenyl group which is unsubstituted or substituted by from one to three substituents; or
A is a group of the Formula (4): 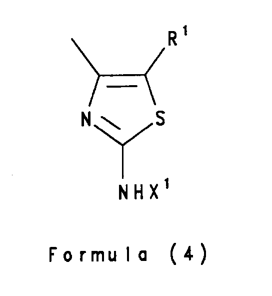
wherein:
R¹ is -H or alkyl; and
X¹ is -H, alkyl or alkylcarbonyl.

3. A benzodifuranone dye of Formula (5): 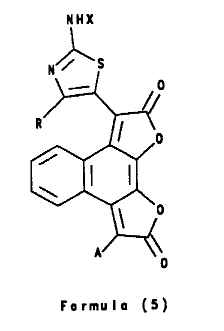 wherein:
R is -H or alkyl;
X is -H, alkyl or alkylcarbonyl;
A is a phenyl group which is unsubstituted or substituted by from one to three substituents; or
A is a group of the Formula (4): 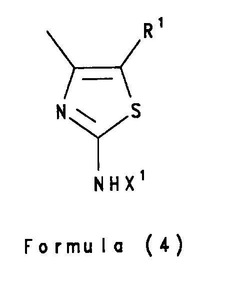
wherein:
R¹ is -H or alkyl; and
X¹ is -H, alkyl or alkylcarbonyl.
